# EUROPÄISCHE PATENTSCHRIFT

(11) **EP 1 453 422 B1**
(45) Veröffentlichungstag und Bekanntmachung des Hinweises auf die Patenterteilung: **06.07.2005**
(21) Anmeldenummer: 02779542.6
(22) Anmeldetag: 09.11.2002
(51) Int. Cl.: A61B 17/04, A61B 17/68

(54) **CHIRURGISCHE VORRICHTUNG ZUM ZUSAMMENSCHIEBEN EINER FADENSCHLAUFE**
SURGICAL DEVICE FOR PUSHING A THREAD LOOP TOGETHER
DISPOSITIF CHIRURGICAL DE DEPLACEMENT D'UNE BOUCLE DE FIL

(30) Priorität: 15.12.2001 DE 10161724
(43) Veröffentlichungstag der Anmeldung: 08.09.2004
(73) Patentinhaber: Aesculap AG & Co. KG, 78532 Tuttlingen (DE)
(72) Erfinder: NESPER, Markus, 78532 Tuttlingen (DE); STEINHILPER, Klaus-Dieter, 78532 Tuttlingen (DE); WEISSHAUPT, Dieter, 78194 Immendingen (DE)
(74) Vertreter: Böhme, Ulrich, Dr. Dipl.-Phys.
(86) Internationale Anmeldenummer: PCT/EP2002/012537
(87) Internationale Veröffentlichungsnummer: WO 2003/051204

(56) Entgegenhaltungen:
- EP-A- 0 602 757
- EP-A- 0 628 286
- DE-A- 2 804 070
- DE-C- 912 619
- DE-U- 29 919 090
- US-A- 4 966 600
- US-A- 5 021 059
- US-A- 5 242 459
- US-A- 5 919 205
- US-A- 6 096 058

## Beschreibung

Die Erfindung betrifft eine chirurgische Vorrichtung zum Zusammenschieben einer Schlaufe eines Fadens, die durch einen längs des Fadens verschiebbaren Rutschknoten des distalen freien Endes des Fadens gebildet wird, mit einem auf dem Faden in Richtung auf die Schlaufe verschiebbaren Schiebeglied, wobei mit dem Schiebeglied oder mit dem am der Schlaufe abgewandten Ende des Schiebegliedes angeordneten proximalen Abschnitt des Fadens ein Griffelement verbunden ist, welches beim Überschreiten einer bestimmten Verschiebekraft die Verbindung zwischen sich und dem Schiebeglied bzw. dem proximalen Abschnitt des Fadens löst.

Im chirurgischen Bereich werden in großem Umfange Fäden eingesetzt, um Körperteile miteinander zu verbinden, Ligaturen zu legen oder Implantatteile untereinander oder mit dem Körper eines Patienten zu verbinden. Dabei ist es üblich, den Faden an seinem distalen, freien Ende in Form einer Schlaufe auszubilden und das freie Ende mit dem anschließenden Fadenabschnitt mittels eines Rutschknotens zu verbinden, d. h. mittels eines Knotens, der längs des Fadens unter Verengung der Schlaufe verschoben werden kann und der dann in dieser Lage verbleibt und nicht zurückgeschoben werden kann. Derartige Knoten sind beispielsweise als sogenannte Roederknoten bekannt.

Zum Verschieben des Rutschknotens und zum Verengen der Schlaufe ist es bekannt, auf dem Faden ein Schiebeglied anzuordnen, so daß der Chirurg die Schlinge einfach dadurch zusammenziehen kann, daß er den Faden an seinem der Schlaufe abgewandten, proximalen Ende festhält und das Schiebeglied auf dem Faden in Richtung auf die Schlaufe verschiebt, dabei wird der Rutschknoten in Richtung auf die Schlaufe verschoben, und dies führt zu einem Zusammenziehen der Schlaufe.

Bei bestimmten Anwendungsfällen kann es beim Zusammenziehen der Schlaufe zu Schädigungen kommen, wenn die Schlaufe zu fest zugezogen wird. Bei herkömmlichen Vorrichtungen dieser Art war es dem Geschick des Operateurs vorbehalten, die Schlaufe immer nur so weit zuzuziehen, daß derartige Schädigungen vermieden werden, dies ist jedoch mit gewissen Unsicherheiten verbunden.

Aus der US-Patentschrift 5,021,059 ist eine Vorrichtung zum Zusammenschieben einer Schlaufe eines Fadens bekannt mit einem auf dem Faden in Richtung auf die Schlaufe verschiebbaren Schiebeglied. Mit dem am der Schlaufe abgewandten Ende des Schiebegliedes angeordneten proximalen Abschnitt des Fadens ist ein Griffelement verbunden, welches beim Überschreiten einer bestimmten Verschiebekraft die Verbindung zwischen sich und dem proximalen Abschnitt des Fadens löst. Die Lösung erfolgt dabei durch zwei Andruckrollen an den Faden, die beim Überschreiten der Zugkraft den Faden freigeben.

Ausgehend von diesem Stand der Technik ist es Aufgabe der Erfindung, eine gattungsgemäße Vorrichtung so auszubilden, daß mit Sicherheit ein zu festes Zuziehen der Schlaufe vermieden wird.

Diese Aufgabe wird bei einer Vorrichtung der eingangs beschriebenen Art erfindungsgemäß dadurch gelöst, daß die Verbindung eine elastische Rastverbindung ist.

Wenn der Chirurg zum Zusammenziehen der Schlaufe das Schiebeglied in Richtung auf die Schlaufe verschiebt, so erfolgt dies bei einer ersten Variante der Erfindung über ein Griffelement, das mit dem Schiebeglied in Verbindung steht, wobei diese Verbindung gelöst wird, sobald eine bestimmte Verschiebekraft überschritten ist. Dadurch wird vermieden, daß die Schlaufe zu fest zugezogen wird, der Operateur merkt außerdem an der Lösung der Verbindung, daß eine bestimmte maximale Verschiebekraft erreicht ist.

Bei einer weiteren Variante der erfindungsgemäßen Lösung greift das Griffelement an dem distalen Abschnitt des Fadens an, der beim Verschieben der Schiebehülse in jedem Fall vom Operateur gegengehalten werden muß, und auch hier erfolgt eine Lösung dieser Verbindung in dem Augenblick, in dem die maximale Verschiebekraft erreicht ist, so daß ein zu festes Zuziehen der Schlaufe vermieden wird, außerdem wird auch hier der Operateur über das Erreichen der maximalen Verschiebekraft durch das Lösen der Verbindung informiert.

Besonders vorteilhaft ist es, wenn das Schiebeglied eine den Faden zumindest an ihrem distalen Ende eng umgebende Hülse ist.

Es ist weiterhin vorteilhaft, wenn das Griffelement eine das Schiebeglied umgebende Griffhülse ist.

Eine besonders vorteilhafte Ausgestaltung ergibt sich, wenn die Griffhülse auf einem als Hülse ausgebildeten Schiebeglied in Fadenrichtung verschiebbar gelagert ist, die beiden Hülsen sind dann vorzugsweise konzentrisch zueinander ausgebildet und haben einen sehr geringen Platzbedarf.

Bei einer bevorzugten Ausführungsform ist vorgesehen, daß die Verbindung von Griffhülse und Schiebeglied durch ein Paar von an der Innenseite der Griffhülse und der Außenseite des Schiebegliedes angeordneten, elastisch ineinandergreifenden Vor- und Rücksprüngen gebildet wird, die beim Überschreiten einer bestimmten Verschiebekraft außer Eingriff gelangen und eine Relativverschiebung von Griffhülse und Schiebeglied ermöglichen.

Dabei ist es günstig, wenn die Vor- und Rücksprünge aneinanderliegende Aufgleitflächen aufweisen.

Weiterhin kann vorgesehen sein, daß die Verschiebebewegung des Griffelementes relativ zum Schiebeglied nach Lösung der Verbindung durch einen Anschlag begrenzt wird.

Bei einer anderen Ausführungsform ist vorgesehen, daß der proximale Abschnitt des Fadens in einem Halteglied gegen eine Verschiebung in proximaler Richtung gesichert angeordnet ist und daß die beim Überschreiten einer Verschiebekraft lösbare Verbindung zwischen dem Halteglied und dem Griffelement besteht. Bei dieser Ausgestaltung hält der Operateur also einerseits das Schiebeglied und andererseits das Griffelement und zieht diese zum Zuziehen der Schlaufe auseinander, bis die Verschiebekraft überschritten wird, dann löst sich das Griffelement von dem proximalen Abschnitt des Fadens und verhindert so eine zu starke Spannung.

Das Halteglied kann insbesondere eine den proximalen Abschnitt des Fadens umgebende Hülse sein.

Weiterhin ist es vorteilhaft, wenn das Griffelement eine das Halteglied umgebende Griffhülse ist.

Eine günstige Ausgestaltung ergibt sich dann, wenn die Griffhülse auf einem als Hülse ausgebildeten Halteglied in Fadenrichtung verschiebbar gelagert ist.

Bei einer besonders bevorzugten Ausführungsform ist vorgesehen, daß die Verbindung von Griffhülse und Halteglied durch ein Paar von an der Innenseite der Griffhülse und an der Außenseite des Haltegliedes angeordneten, elastisch ineinandergreifenden Vor- und Rücksprüngen gebildet wird, die beim Überschreiten einer bestimmten Verschiebekraft außer Eingriff gelangen und eine Relativverschiebung von Griffhülse und Halteglied ermöglichen.

Insbesondere können die Vor- und Rücksprünge dabei aneinanderliegende Aufgleitflächen aufweisen.

Es ist auch vorteilhaft, wenn die Verschiebebewegung des Griffelementes relativ zum Halteglied nach Lösung der Verbindung durch einen Anschlag begrenzt wird.

Bei einer besonders bevorzugten Ausführungsform ist vorgesehen, daß die Schlaufe durch zwei mittels der Schlaufe gegeneinander spannbare Implantatteile geführt sind. Man erhält somit eine bereits vorkonfektionierte Vorrichtung, bei der zwei durch die Schlaufe gegeneinander zu spannende Implantatteile bereits Teil der Vorrichtung sind, diese Vorrichtung umfaßt den Faden mit der Schlaufe, die daran gehaltenen Implantatteile und das Schiebeglied sowie ein erfindungsgemäßes Griffelement mit lösbarer Verbindung. Diese gesamte vorkonfektionierte Einheit dient der Anlage und dem Spannen der beiden Implantatteile und kann als Einmalpackung ausgebildet sein.

Insbesondere kann vorgesehen sein, daß die Implantatteile einander zugewandte plattenförmige Anlageflächen aufweisen, die von gegenüberliegenden Seiten her an die Außenflächen von zwei nebeneinander angeordneten Knochenteilen anlegbar sind, beispielsweise können diese Knochenteile durch den Schädelknochen einerseits und durch eine in eine Öffnung des Schädelknochens einzusetzende Knochenplatte andererseits gebildet werden.

Die nachfolgende Beschreibung bevorzugter Ausführungsformen der Erfindung dient im Zusammenhang mit der Zeichnung der näheren Erläuterung. Es zeigen:
- Figur 1:: eine schematische Längsschnittansicht einer Vorrichtung zum Zusammenschieben einer Fadenschlaufe mit einem hülsenförmigen, ein Schiebeglied umgebenden Griffelement in seiner Vorschiebestellung;
- Figur 2:: eine Ansicht ähnlich Figur 1 nach Lösung der Verbindung zwischen Griffelement und Schiebeglied;
- Figur 3:: eine Längsschnittansicht durch ein abgewandeltes Ausführungsbeispiel einer Vorrichtung zum Zusammenschieben einer Fadenschlaufe mit einem ein Fadenhalteglied umgebenden hülsenförmigen Griffelement bei bestehender Verbindung zwischen Griffelement und Halteglied und
- Figur 4:: eine Ansicht ähnlich Figur 3 nach Lösung der Verbindung zwischen Griffelement und Halteglied.

Die in der Zeichnung dargestellte Vorrichtung umfaßt einen Faden 1, beispielsweise einen dünnen Kunststoffaden, der an einem Ende, dem proximalen Ende 2 mit einem Halteglied 3 in Form eines Ringes fest verbunden ist, während das andere, distale Ende 4 durch Öffnungen 5, 6 von zwei scheibenförmigen Implantaten 7, 8 so hindurchgesteckt ist, daß dieses distale Ende 4 zunächst durch beide Implantate 7, 8 hindurchgeführt, dann an der Außenseite des einen Implantates 8 entlang - und wieder durch die beiden Implantate 7, 8 zurückgeführt ist und somit eine Schlaufe 9 ausbildet, die dadurch geschlossen ist, daß das distale Ende 4 des Fadens 1 mit dem Faden 1 selbst durch einen Knoten 10 verbunden ist. Dieser Knoten 10 ist als Schiebeknoten ausgebildet, beispielsweise in Form eines an sich bekannten Roederknotens, so daß der Knoten 10 in Richtung auf die Schlaufe 9 relativ zum Faden 1 verschiebbar ist, wobei die Schlaufe 9 zusammengezogen wird, während eine Verschiebung des Knotens 10 in der entgegengesetzten Richtung nicht möglich ist.

Der Faden 1 läuft zwischen der Schlaufe 9 einerseits und dem Halteglied 3 andererseits durch ein Schiebeglied 11 in Form einer Hülse hindurch, diese Hülse ist an ihrem der Schlaufe 9 zugewandten distalen Ende 12 konisch ausgebildet und weist eine sich in proximaler Richtung erweiternde und das gesamte Schiebeglied 11 durchsetzende Durchgangsöffnung 13 auf, die den Faden 1 im Bereich des distalen Endes 12 des Schiebegliedes 11 eng anliegend umgibt und die längs des Fadens 1 frei verschieblich ist.

Auf dem hülsenförmigen Schiebeglied 11 ist koaxial zu dem Schiebeglied 11 ein ebenfalls hülsenförmig ausgebildetes Griffelement 14 in Richtung des Fadens 1 verschiebbar gelagert, welches an seinem der Schlaufe 9 zugewandten distalen Ende 15 eine flanschförmige Verbreiterung 16 trägt.

An seiner Innenwand 17 weist das hülsenförmige Griffelement 14 nach innen vorstehende Vorsprünge 18 auf, die in komplementäre Vertiefungen 19 in der Außenwand 20 des hülsenförmigen Schiebegliedes 11 einrasten können, so daß dadurch einer Verschiebung des Griffelementes 14 relativ zum Schiebeglied 11 ein Widerstand entgegengesetzt wird.

Im Bereich der Vertiefungen 19 ist die Wand des hülsenförmigen Schiebegliedes 11 in Form von Längsschlitzen 21 durchbrochen, so daß die Wand geringfügig elastisch radial nach innen biegbar ist. Außerdem weisen Vorsprünge 18 und Vertiefungen 19 seitliche, schräg verlaufende Anlageflächen 22 und 23 auf.

In distaler Richtung schließen sich an die Vertiefungen 19 achsparallele Längsnuten 24 an, die in einem Abstand von den Vertiefungen 19 in einer nach außen vorspringenden Stufe 25 enden.

Die beschriebene Vorrichtung aus Faden 1, Schiebeglied 11 und Griffelement 14 sowie den beiden scheibenförmigen Implantaten 7, 8 bilden bei dem dargestellten Ausführungsbeispiel eine vorgefertigte Einheit aus, die in dieser Form dem Operateur in sterilem Zustand zur Verfügung gestellt wird. Es werden dabei sterilisierbare Kunststoffmaterialien eingesetzt, der Faden 1 und die Implantate 7 und 8 können aus resorbierbaren Kunststoffmaterialien bestehen.

Diese Vorrichtung kann beispielsweise, wie in der Zeichnung gezeigt, zur Fixierung von zwei nebeneinander angeordneten Knochenplatten 26, 27 dienen, beispielsweise zur Fixierung eines Knochendeckels in einer Öffnung des Schädelknochens. Zu diesem Zweck werden die scheibenförmigen Implantate 7, 8 relativ zu den beiden Knochenplatten 26 und 27 so angeordnet, daß ein Implantat 7 an einer Seite der beiden Knochenplatten 26, 27 zur Anlage kommt, das andere Implantat 8 an der anderen Seite dieser Knochenplatten 26 und 27, so daß die Knochenplatten 26 und 27 jeweils zwischen die beiden scheibenförmigen Implantate 7, 8 eingreifen. Im Spalt 28 zwischen der Knochenplatte 26 und der Knochenplatte 27 verläuft die Schlaufe 9 des Fadens 1, wobei sich die Schlaufe 9 vorzugsweise in Längsrichtung dieses Spaltes 28 erstreckt. In der Zeichnung ist diese Schlaufe gegenüber der Längsrichtung des Spaltes 28 gedreht, um die Schlaufe besser zeigen zu können, dies ist aber nur bei relativ breiten Spalten 28 möglich, wenn der Spalt schmaler ist, ist es vorteilhaft, die Schlaufe in die Ebene der Spaltlängsrichtung zu drehen.

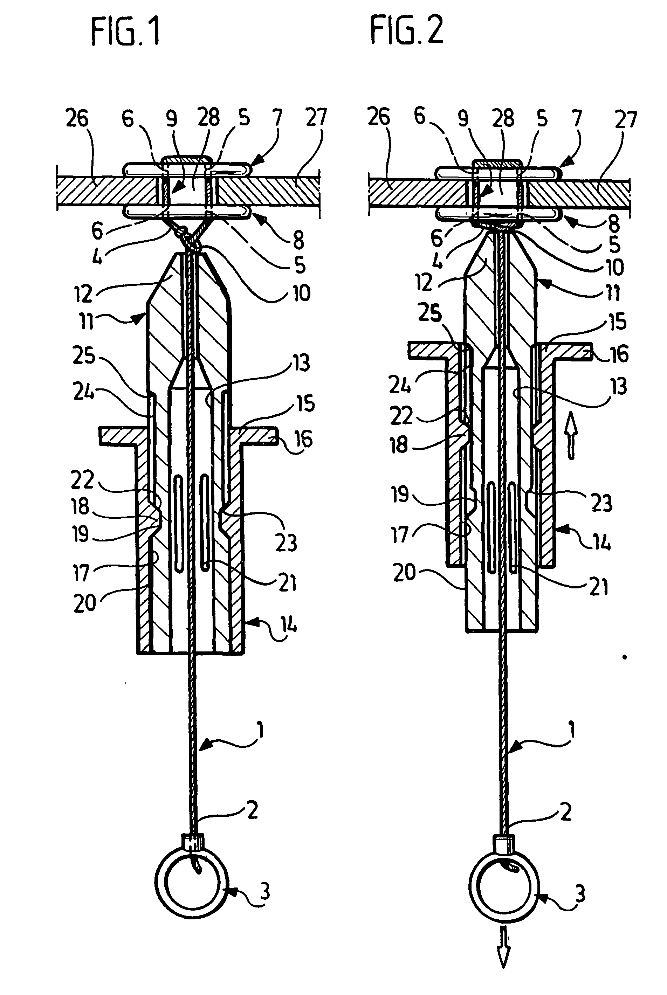

Die vorgefertigte Vorrichtung wird so geliefert, daß das Schiebeglied 11 und das Griffelement 14 in axialer Richtung relativ zueinander festgelegt sind, dies wird dadurch erreicht, daß die Vorsprünge 18 in die Vertiefungen 19 eintauchen, wie dies in Figur 1 dargestellt ist.

Nach dem Anlegen der Implantate 7, 8 an die Knochenplatten 26 und 27 verschiebt der Operateur das Schiebeglied 11 mit Hilfe des Griffelementes 14 in Richtung auf die Schlaufe 9 und hält dabei das proximale Ende 2 des Fadens 1 mittels des Haltegliedes 3 fest, mit anderen Worten es werden Griffelement 14 und Schiebeglied 11 einerseits und Halteglied 3 andererseits auseinandergezogen. Dadurch wird der Knoten 10 in Richtung auf die Schlaufe 9 verschoben und die Schlaufe 9 wird zugezogen. Sie spannt dabei die beiden scheibenförmigen Implantate 7, 8 gegeneinander und fixiert so die dazwischenliegenden Knochenplatten 26 und 27 relativ zueinander.

Beim Vorschieben des Knotens 10 wird die Widerstandskraft beim Spannen immer größer, und schließlich ist die Verschiebekraft, die der Operateur über das Griffelement 14 auf das Schiebeglied 11 ausübt, so groß, daß ein bestimmter Maximalwert überschritten wird. Dies führt zu einer elastischen Verformung im Bereich der Außenwand 20 des Schiebegliedes 11, dieser Wandbereich wird etwas radial nach innen verformt. Dadurch können die Vorsprünge 18 aus den Vertiefungen 19 austreten, dieses Austreten wird durch die schrägen Anlageflächen 22 und 23 unterstützt. Nach dem Austreten gelangen die Vorsprünge 18 in die Längsnuten 24 des Schiebegliedes 11, und dies ermöglicht es dem Griffelement 14, relativ zum Schiebeglied 11 verschoben zu werden, und zwar so weit, bis die Vorsprünge 18 an der Stufe 25 anschlagen.

Einerseits wird durch dieses Ausrasten der Vorsprünge 18 aus den Vertiefungen 19 das Vorschieben des Knotens 10 und damit das Spannen der Schlaufe 9 unterbrochen, andererseits spürt der Operateur an diesem Ausrasten sofort, daß der Maximalwert der Verschiebekraft erreicht ist, er kann damit den Spannvorgang der Schlaufe 9 abbrechen. Die Anlage der Implantate 7, 8 ist beendet. Der Faden 1 kann unmittelbar anschließend an den Knoten 10 abgeschnitten werden, die beiden scheibenförmigen Implantate 7, 8 bleiben durch die gespannte Schlaufe 9 zusammengespannt im Körper und fixieren die beiden Knochenplatten 26 und 27 relativ zueinander.

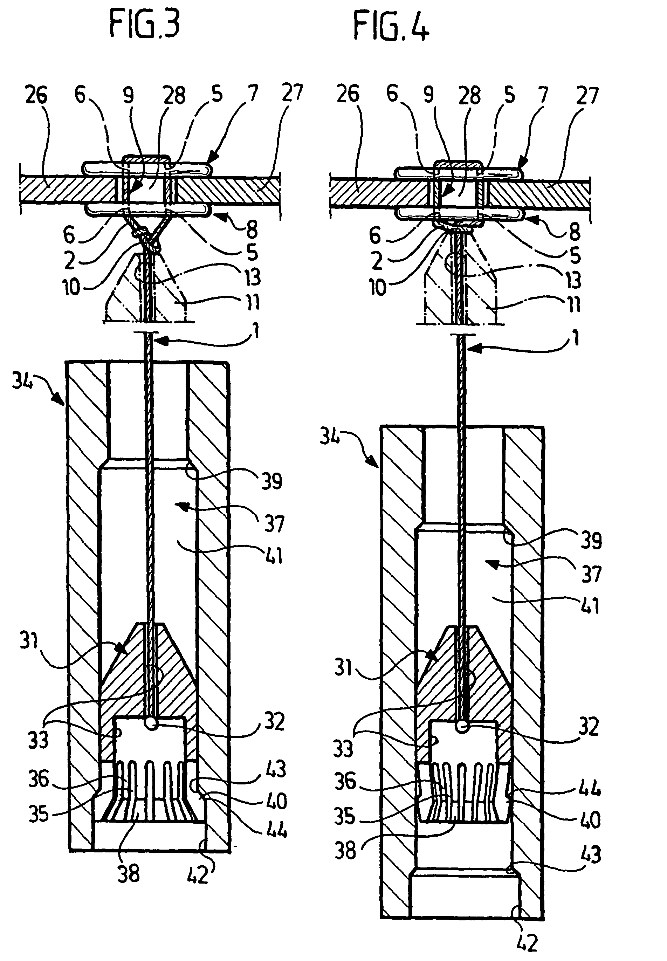

Die in den Figuren 3 und 4 dargestellte Ausführungsform einer Vorrichtung zum Spannen einer Fadenschlaufe ist ähnlich aufgebaut wie die der Figuren 1 und 2, einander entsprechende Teile tragen daher dieselben Bezugszeichen.

Das Schiebeglied 11 ist bei dieser Ausführungsform einfacher aufgebaut, es weist nämlich kein relativ zum Schiebeglied 11 verschiebbares Griffelement 14 auf, sondern das Schiebeglied 11 ist gleichzeitig auch ein Griffelement zum Vorschieben des Knotens 10. Dieses Schiebeglied 11 ist in der Darstellung der Figur 3 und 4 durch strichpunktierte Linien lediglich angedeutet.

Das ringförmige Halteglied 3 beim Ausführungsbeispiel der Figuren 1 und 2 ist beim Ausführungsbeispiel der Figuren 3 und 4 ersetzt durch ein hülsenförmiges Halteglied 31 mit einer sich in proximaler Richtung stufenförmig erweiternden Durchgangsöffnung 33. Durch den distalen Teil dieser Durchgangsöffnung 33 ist das proximale Ende 4 des Fadens 1 hindurchgeführt, der Faden 1 wird in diesem Bereich von der Durchgangsöffnung 33 dicht umgeben und trägt an seinem aus diesem engen Abschnitt der Durchgangsöffnung 33 herausragenden Ende ein verbreitertes, im dargestellten Ausführungsbeispiel kugelförmiges Anschlagelement 32, welches die Verschiebung des Fadens 1 in distaler Richtung dadurch verhindert, daß das Anschlagelement 32 an der Stufe der Durchgangsöffnung 33 zur Anlage kommt.

Am proximalen Ende ist die Außenwand des Haltegliedes 31 durch Längseinschnitte 35 in nebeneinanderliegende Zungen 36 unterteilt, die an ihrem proximalen, freien Ende über den Umfang des hülsenförmigen Haltegliedes nach außen hervorstehen und dadurch jeweils einen radial vorstehenden Vorsprung 38 ausbilden.

Das hülsenförmige Halteglied 31 ist in einem hülsenförmigen Griffelement 34 in dessen Längsrichtung verschieblich aufgenommen. Der Innenraum 37 dieses hülsenförmigen Griffelementes 34 verbreitert sich in proximaler Richtung an zwei Stufen 39 und 40, der Durchmesser des zwischen den beiden Stufen 39 und 40 gelegenen Abschnittes 41 entspricht dabei dem Außendurchmesser des hülsenförmigen Haltegliedes 31, das in diesem mittleren Abschnitt 41 verschiebbar geführt ist.

Die Zungen 36 liegen dabei mit ihren Vorsprüngen 38 in dem proximalen Abschnitt 42 des Innenraums des hülsenförmigen Griffelementes 34 und hintergreifen die Stufe 40 zwischen dem mittleren Abschnitt 41 und dem proximalen Abschnitt 42, so daß dadurch das Halteglied 31 gegen ein Einschieben in distaler Richtung zurückgehalten wird.

Allerdings läßt sich das Halteglied 31 dann weiter in distaler Richtung in den mittleren Abschnitt 41 einschieben, wenn beim Überschreiten einer bestimmten Zugkraft, die durch den Faden 1 auf das Halteglied 31 ausgeübt wird, die Zungen 36 elastisch radial nach innen gebogen werden, dann können nämlich die Vorsprünge 38 an der Stufe 40 aufgleiten. Um dies zu erleichtern, sind die Vorsprünge 38 und die Stufe 40 mit schrägen Anlageflächen 43, 44 versehen.

Die Einschubtiefe des Haltegliedes 31 in den mittleren Abschnitt 41 des Griffelementes 34 wird dadurch begrenzt, daß das Halteglied 31 an der Stufe 39 anschlägt. Es wäre aber auch ohne weiteres möglich, die Stufe 39 wegzulassen, so daß beim Überschreiten der maximalen Verschiebekraft einfach das Griffelement 34 vollständig vom Halteglied 31 abgezogen würde.

Bei der Verwendung der beschriebenen Vorrichtung wird ähnlich vorgegangen wie bei der Vorrichtung der Figuren 1 und 2. Durch Auseinanderschieben des Schiebegliedes 11 einerseits und des Griffelementes 34 andererseits wird die Schlaufe 9 gespannt, bis ein maximaler Wert der Verschiebekraft erreicht ist. Sobald dies der Fall ist, wird die axiale Festlegung des Haltegliedes 31 in dem hülsenförmigen Griffelement 34 aufgehoben, und bei einem weiteren Entfernen des Griffelementes 34 vom Schiebeelement 11 wird nur das Halteglied 31 tiefer in das Griffelement 34 eingeschoben, ohne daß das proximale Ende 2 des Fadens 1 weiter gespannt wird, d. h. das Spannen der Schlaufe 9 wird beendet. Gleichzeitig spürt der Operateur durch die plötzliche Verschiebung des Griffelementes 34 und durch das Vorbeigleiten der Vorsprünge 38 an der Stufe 40, daß die maximale Zugkraft erreicht ist und daß der Spannvorgang damit beendet ist.

Während also bei dem Ausführungsbeispiel der Figuren 1 und 2 eine lösbare Verbindung im Bereich zwischen einem Griffelement und einem Schiebeglied realisiert ist, zeigt das Ausführungsbeispiel der Figuren 3 und 4 eine derartige lösbare Verbindung im Bereich zwischen einem Griffelement und dem Halteglied für das proximale Ende des Fadens.

## Patentansprüche

1. Chirurgische Vorrichtung zum Zusammenschieben einer Schlaufe (9) eines Fadens (1), die durch einen längs des Fadens (1) verschiebbaren Rutschknoten des distalen freien Endes des Fadens (1) gebildet wird, mit einem auf dem Faden (1) in Richtung auf die Schlaufe (9) verschiebbaren Schiebeglied (11), wobei mit dem Schiebeglied (11) oder mit dem am der Schlaufe (9) abgewandten Ende des Schiebegliedes angeordneten proximalen Abschnitt (2) des Fadens (1) ein Griffelement (14; 34) verbunden ist, welches beim Überschreiten einer bestimmten Verschiebekraft die Verbindung zwischen sich und dem Schiebeglied (11) bzw. dem proximalen Abschnitt (2) des Fadens (1) löst, **dadurch gekennzeichnet, daß** die Verbindung eine elastische Rastverbindung (18, 19, 21; 36, 38, 40) ist.

2. Vorrichtung nach Anspruch 1, **dadurch gekennzeichnet, daß** das Schiebeglied (11) eine den Faden zumindest an ihrem distalen Ende (12) eng umgebende Hülse ist.

3. Vorrichtung nach einem der Ansprüche 1 oder 2, **dadurch gekennzeichnet, daß** das Griffelement (14) eine das Schiebeglied (11) umgebende Griffhülse ist.

4. Vorrichtung nach Anspruch 3, **dadurch gekennzeichnet, daß** die Griffhülse auf einem als Hülse ausgebildeten Schiebeglied (11) in Fadenrichtung verschiebbar gelagert ist.

5. Vorrichtung nach Anspruch 4, **dadurch gekennzeichnet, daß** die Verbindung von Griffhülse (14) und Schiebeglied (11) durch ein Paar von an der Innenseite der Griffhülse (14) und der Außenseite des Schiebegliedes (11) angeordneten, elastisch ineinandergreifenden Vor- und Rücksprüngen (18, 19) gebildet wird, die beim Überschreiten einer bestimmten Schiebekraft außer Eingriff gelangen und eine Relativverschiebung von Griffhülse (14) und Schiebeglied (11) ermöglichen.

6. Vorrichtung nach Anspruch 5, **dadurch gekennzeichnet, daß** die Vor- und Rücksprünge (18, 19) aneinanderliegende Aufgleitflächen (22, 23) aufweisen.

7. Vorrichtung nach einem der Ansprüche 3 bis 6, **dadurch gekennzeichnet, daß** die Verschiebebewegung des Griffelementes (14) relativ zum Schiebeglied (11) nach Lösung der Verbindung durch einen Anschlag (25) begrenzt wird.

8. Vorrichtung nach einem der Ansprüche 1 oder 2, **dadurch gekennzeichnet, daß** der proximale Abschnitt (2) des Fadens (1) in einem Halteglied (31) gegen eine Verschiebung in proximaler Richtung gesichert angeordnet ist und daß die beim Überschreiten einer Verschiebekraft lösbare Verbindung zwischen dem Halteglied (31) und dem Griffelement (34) besteht.

9. Vorrichtung nach Anspruch 8, **dadurch gekennzeichnet, daß** das Halteglied (31) eine den proximalen Abschnitt (2) des Fadens (1) umgebende Hülse ist.

10. Vorrichtung nach einem der Ansprüche 8 oder 9, **dadurch gekennzeichnet, daß** das Griffelement (34) eine das Halteglied (31) umgebende Griffhülse ist.

11. Vorrichtung nach Anspruch 10, **dadurch gekennzeichnet, daß** die Griffhülse (34) auf einem als Hülse ausgebildeten Halteglied (31) in Fadenrichtung verschiebbar gelagert ist.

12. Vorrichtung nach Anspruch 11, **dadurch gekennzeichnet, daß** die Verbindung von Griffhülse (34) und Halteglied (31) durch an der Innenseite der Griffhülse (34) und an der Außenseite des Haltegliedes (31) angeordnete, elastisch ineinandergreifende Vor- und Rücksprünge (38, 40) gebildet wird, die beim Überschreiten einer bestimmten Verschiebekraft außer Eingriff gelangen und eine Relativverschiebung von Griffhülse (34) und Halteglied (31 ermöglichen.

13. Vorrichtung nach Anspruch 12, **dadurch gekennzeichnet, daß** die Vor- und Rücksprünge (38, 40) aneinanderliegende Aufgleitflächen (43, 44) aufweisen.

14. Vorrichtung nach einem der Ansprüche 8 bis 13, **dadurch gekennzeichnet, daß** die Verschiebebewegung des Griffelementes (34) relativ zum Halteglied (31) nach Lösung der Verbindung durch einen Anschlag (39) begrenzt wird.

15. Vorrichtung nach einem der voranstehenden Ansprüche, **dadurch gekennzeichnet, daß** eine Schlaufe (9) durch zwei mittels der Schlaufe gegeneinander spannbare Implantatteile (7, 8) sowie durch die Vorrichtung geführt ist.

16. Vorrichtung nach Anspruch 15, **dadurch gekennzeichnet, daß** die Implantatteile (7, 8) einander zugewandte plattenförmige Anlageflächen aufweisen, die von gegenüberliegenden Seiten her an die Außenflächen von zwei nebeneinander angeordneten Knochenteilen (26, 27) anlegbar sind.

## Claims

1. Surgical device for pushing together a loop (9) of a thread (1) which is formed by a slip knot at the distal free end of the thread (1) which can be displaced along the thread (1), with a displacing element (11) which can be displaced on the thread (1) in the direction of the loop (9), wherein the displacing element (11) or the proximal portion (2) of the thread (1) disposed at the end of the displacing element remote from the loop (9) has connected to it a gripping element (14; 34) which releases the connection between itself and the displacing element (11) or the proximal portion (2) of the thread (1) when a specific displacing force is exceeded, **characterised in that** the connection is a flexible latching connection (18, 19, 21; 36, 38, 40) .

2. Device according to Claim 1, **characterized in that** the displacing element (11) is a sleeve closely surrounding the thread, at least at its distal end (12).

3. Device according to either of Claims 1 and 2, **characterized in that** the gripping element (14) is a gripping sleeve surrounding the displacing element (11).

4. Device according to claim 3, **characterized in that** the gripping sleeve is mounted displaceably in the direction of the thread on a displacing element (11) formed as a sleeve.

5. Device according to Claim 4, **characterized in that** the connection of the gripping sleeve (14) and the displacing element (11) is formed by a pair of projections and indentations (18, 19) which are disposed on the inner aide of the gripping sleeve (14) and the outer side of the displacing element (11), engage flexibly in one another, disengage when a specific displacing force is exceeded and permit a relative displacement of the gripping sleeve (14) and the displacing element (11).

6. Device according to Claim 5, **characterized in that** the projections and indentations (18, 19) have slide-on surfaces (22, 23) which lie against one another.

7. Device according to one of Claims 3 to 6, **characterized in that** the displacing movement of the gripping element (14) in relation to the displacing element (11) is limited after release of the connection by a stop (25).

8. Device according to either of Claims 1 and 2, **characterized in that** the proximal portion (2) of the thread (1) is disposed in a holding element (31) in such a way that it is secured against displacement in the proximal direction and **in that** the connection that is releasable when a displacing force is exceeded is between the holding element (31) and the gripping element (34).

9. Device according to Claim 8, **characterized in that** the holding element (31) is a sleeve surrounding the proximal portion (2) of the thread (1).

10. Device according to either of Claims 8 and 9, **characterised in that** the gripping element (34) is a gripping sleeve surrounding the holding element (31).

11. Device according to Claim 10, **characterized in that** the gripping sleeve (34) is mounted displaceably in the direction of the thread on a holding element (31) formed as a sleeve.

12. Device according to Claim 11, **characterized in that** the connection of the gripping sleeve (34) and the holding element (31) is formed by projections and indentations (38, 40) which are disposed on the inner side of the gripping sleeve (34) and the outer side of the holding element (31), engage flexibly in one another, disengage when a specific displacing force is exceeded and permit a relative displacement of the gripping sleeve (34) and the holding element (31).

13. Device according to Claim 12, **characterized in that** the projections and indentations (38, 40) have slide-on surfaces (43, 44) which lie against one another.

14. Device according to one of Claims B to 13, **characterized in that** the displacing movement of the gripping element (34) in relation to the holding element (31) is limited after release of the connection by a stop (39).

15. Device according co one of the preceding claims, **characterized in that** a loop (9) is led through two implant parts (7, 8), which can be braced against each other by means of the loop, and through the device.

16. Device according to Claim 15, **characterized in that** the implant parts (7, 8) have mutually facing plate-shaped butting surfaces, which can be placed from opposite sides against the outer surfaces of two bone parts (26, 27) disposed next to each other.

## Revendications

1. Dispositif chirurgical pour faire coulisser une boucle (9) d'un fil (1), qui est formée par un noeud coulant de l'extrémité distale libre du fil (1) déplaçable le long du fil (1), avec un élément coulissant (11) déplaçable sur le fil (1) dans la direction de la boucle (9), dans lequel un élément de prise (14 ; 34), est relié à l'élément coulissant (11) ou au tronçon proximal (2) du fil (1) disposé à l'extrémité de l'élément coulissant (11) dirigée à l'opposé de la boucle (9), lequel élément de prise, en cas de dépassement d'une force de déplacement déterminée, détache la liaison entre lui-même et l'élément coulissant (11) ou le tronçon proximal (2) du fil (1), **caractérisé en ce que** la liaison est une liaison élastique à crans (18, 19, 21 ; 36, 38, 40).

2. Dispositif selon la revendication 1, **caractérisé en ce que** l'élément coulissant (11) est un manchon entourant étroitement le fil au moins à son extrémité distale (12).

3. Dispositif selon l'une quelconque des revendications 1 ou 2, **caractérisé en ce que** l'élément de prise (14) est un manchon de prise entourant l'élément coulissant (11).

4. Dispositif selon la revendication 3, **caractérisé en ce que** le manchon de prise est logé de manière déplaçable dans la direction du fil sur un élément coulissant (11) configuré comme un manchon.

5. Dispositif selon la revendication 4, **caractérisé en ce que** la liaison entre le manchon de prise (14) et l'élément coulissant (11) est formée par une paire de parties en saillie et de parties en retrait (18, 19) s'engageant élastiquement l'une dans l'autre, disposées sur la face intérieure du manchon de prise (14) et sur la face extérieure de l'élément coulissant (11), qui, en cas de dépassement d'une force de poussée déterminée, se désengagent l'une de l'autre et permettent un déplacement relatif du manchon de prise (14) et de l'élément coulissant (11).

6. Dispositif selon la revendication 5, **caractérisé en ce que** les parties en saillie et les parties en retrait (18, 19) comportent des surfaces de glissement (22, 23) placées les unes à côté des autres.

7. Dispositif selon l'une quelconque des revendications 3 à 6, **caractérisé en ce que** le déplacement du manchon de prise (14) par rapport à l'élément coulissant (11) après le détachement de la liaison est limité par une butée (25).

8. Dispositif selon l'une quelconque des revendications 1 ou 2, **caractérisé en ce que** le tronçon proximal (2) du fil (1) est disposé dans un élément de retenue (31) de manière protégée contre un déplacement dans la direction proximale et **en ce que** la liaison détachable en cas de dépassement d'une force de poussée existe entre l'élément de retenue (31) et l'élément de prise (34).

9. Dispositif selon la revendication 8, **caractérisé en ce que** l'élément de retenue (31) est un manchon entourant le tronçon proximal (2) du fil (1).

10. Dispositif selon l'une quelconque des revendications 8 ou 9, **caractérisé en ce que** l'élément de prise (34) est un manchon de prise entourant l'élément de retenue (31).

11. Dispositif selon la revendication 10, **caractérisé en ce que** le manchon de prise (34) est logé de manière déplaçable dans la direction du fil sur un élément de retenue (31) configuré comme un manchon.

12. Dispositif selon la revendication 11, **caractérisé en ce que** la liaison entre le manchon de prise (34) et l' élément de retenue (31) est formée par des parties en saillie et des parties en retrait (38, 40) s'engageant élastiquement l'une dans l'autre, disposées sur la face intérieure du manchon de prise (34) et sur la face extérieure de l'élément de retenue (31), qui, en cas de dépassement d'une force de poussée déterminée, se désengagent l'une de l'autre et permettent un déplacement relatif du manchon de prise (34) et de l'élément de retenue (31).

13. Dispositif selon la revendication 12, **caractérisé en ce que** les parties en saillie et les parties en retrait (38, 40) comportent des surfaces de glissement (43, 44) placées les unes à côté des autres.

14. Dispositif selon l'une quelconque des revendications 8 à 13, **caractérisé en ce que** le déplacement du manchon de prise (34) par rapport à l'élément de retenue (31) après le détachement de la liaison est limité par une butée (39).

15. Dispositif selon l'une quelconque des revendications précédentes, **caractérisé en ce qu'**une boucle (9) est guidée par deux parties d'implant (7, 8), qui peuvent être serrées l'une contre l'autre à l'aide de la boucle, ainsi que par le dispositif.

16. Dispositif selon la revendication 15, **caractérisé en ce que** les parties d'implant (7, 8) comportent des surfaces d'appui en forme de plaque tournées les unes vers les autres qui, à partir de côtés opposés, peuvent être placées sur les faces extérieures de deux parties d'os (26, 27) placées l'une à côté de l'autre.
